# EUROPEAN PATENT SPECIFICATION

(11) **EP 1 136 490 B1**
(45) Date of publication and mention of the grant of the patent: **07.01.2004**
(21) Application number: 01115079.4
(22) Date of filing: 02.02.1996
(51) Int. Cl.: C07D 405/12, A61K 31/4523, A61P 25/00

(54) **Process for preparation of paroxetin hydrochloride anhydrate**
Verfahren zur Herstellung von Paroxetinhydrochloridanhydrat
Procédé pour la préparation de l'hydrochlorure de Paroxètine anhydre

(30) Priority: 06.02.1995 GB 9502297; 17.02.1995 GB 9503112; 15.05.1995 GB 9509807
(43) Date of publication of application: 26.09.2001
(62) Divisional of application: 96903970.0
(73) Proprietor: SmithKline Beecham plc, Brentford, Middlesex TW8 9GS (GB)
(72) Inventor: Jacewicz, Victor Witold, Near Leigh, Tonbridge, Kent TN11 9AN (GB); Ward, Neal, Near Leigh, Tonbridge, Kent TN11 9AN (GB)
(74) Representative: Giddings, Peter John, Dr.

(56) References cited:
- EP-A- 0 223 403
- GB-A- 8 526 407
- P. CHRISTOPHER BUXTON ET AL.: "Solid-State Forms of Paroxetine Hydrochloride" INTERNATIONAL JOURNAL OF PHARMACEUTICS, vol. 42, 1988, pages 135-143, XP000572028 AMSTERDAM NL
- CHRISTOPHER BUXTON P.: 'Solid-state forms of paroxetine hydrochloride' INTERNATIONAL JOURNAL OF PHARMACEUTICS vol. 42, 1998, AMSTERDAM, pages 135 - 143

## Description

The present invention relates to processes for preparing paroxetine hydrochloride anhydrate.

EP-B-223403 (Beecham Group plc) describes paroxetine hydrochloride hemihydrate and its use in treating certain medical disorders. Example 8 in this document describes the preparation of paroxetine hydrochloride anhydrate as platelets melting at 118°C and with IR bands at 890,1200,1490, 3400 and 3640cm⁻¹, by crystallisation from a water-containing solvent. This material is hereinafter referred to as Form Z. Subsequent repetition of the preparation described in Example 8 has failed to yield any type of paroxetine hydrochloride anhydrate, and there is no clear teaching elsewhere in the document of any alternative route or modification to the process which would generate the anhydrate.

Paroxetine hydrochloride anhydrate is also purported to be disclosed in the International Journal of Pharmaceutics 42, (1988) 135 to 143, published by Elsevier. The anhydrate is said to be produced by crystallising paroxetine hydrochloride from anhydrous propan-2-ol. Subsequent repetition of this process has resulted in a propan-2-ol solvate of paroxetine hydrochloride. That is to say that there is bound propan-2-ol in the product. This bound propan-2-ol cannot be removed by conventional drying techniques such as vacuum oven drying.

Paroxetine hydrochloride anhydrate substantially free of bound propan-2-ol, has not been described in the literature, nor has any method been disclosed which would yield such a product as an inevitable result. A method for preparing paroxetine hydrochloride anhydrate substantially free of bound propan-2-ol has now been found.

In first aspect, the present invention provides a process for the preparation of paroxetine hydrochloride anhydrate comprising less than 2 % bound organic solvent which comprises displacing the solvated solvent from a paroxetine hydrochloride solvate using a displacing agent, wherein the solvated solvent is acetone and the displacing agent is water.

Accordingly, the present application describes paroxetine hydrochloride anhydrate substantially free of bound propan-2-ol.

The present application also describes paroxetine hydrochloride anhydrate substantially free of bound organic solvent.

Substantially free of bound organic solvent is to be interpreted to be less than the amount of propan-2-ol which would remain solvated, i.e., bound, within the crystal lattice of the product under conventional vacuum oven drying conditions.

The present application also describes paroxetine hydrochloride solvates other than the propan-2-ol solvate as precursors in the preparation of paroxetine hydrochloride anhydrate substantially free of bound organic solvent. Examples of such solvates include solvates from ketones such as acetone.

Preferably, paroxetine hydrochloride anhydrate substantially free of bound propan-2-ol is provided in substantially pure form. Suitably, paroxetine hydrochloride anhydrate substantially free of bound propan-2-ol is provided with a purity of the paroxetine hydrochloride anhydrate - of greater than 50%, preferably greater than 60%, more preferably greater than 70%, yet more preferably greater than 80% and even more preferably greater than 90%. Most preferably the paroxetine hydrochloride anhydrate is provided in substantially pure form, i.e., paroxetine hydrochloride anhydrate substantially free of bound propan-2-ol is greater than 95% pure.

It should be understood that the paroxetine hydochloride anhydrate substantially free of bound propan-2-ol may contain unbound water that is to say water which is other than water of crystallisation.

Typically the amount of bound organic solvent on a weight for weight basis would be less than 2.0%, preferably less than 1.8%, more preferably less than 1.5%, even more preferably less than 1.0%, yet more preferably less than 0.5% and most preferably less than 0.1%.

Generally, all percentages indicated herein are on a weight for weight basis unless otherwise stated.

One form of paroxetine hydrochloride anhydrate substantially free of bound propan-2-ol or substantially free of bound organic solvent is paroxetine hydrochloride anhydrate in Form A, (as hereinafter defined).

This form of paroxetine hydrochloride anhydrate may be distinguished from the material formed as a result of carrying out the procedures mentioned in EP-B-0223403 and the International Journal of Pharmaceutics 42, (1988), 135 to 143, by crystalline shape, solvent analysis or techniques such as IR, melting point, X-ray diffraction, NMR, DSC, microscopy and any other analytical techniques which differentiate one form from another.

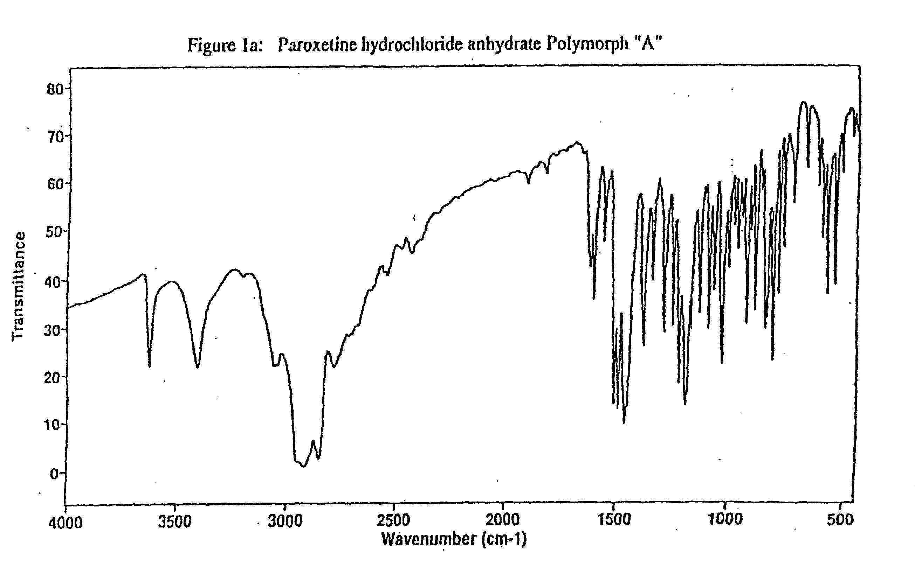

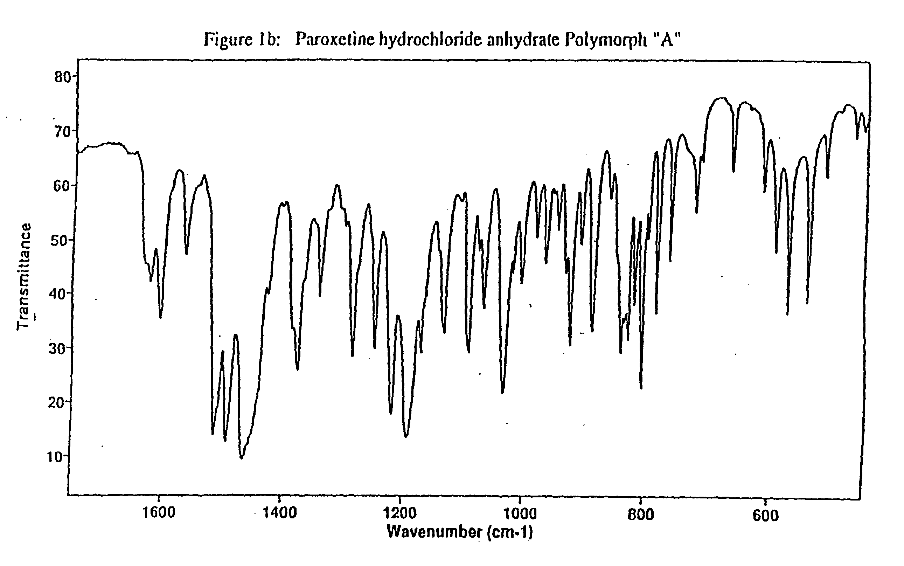

For example, Form A substantially free of solvent may be distinguished from other forms by the following analytical data. Form A has a melting point of about 123-125°C when obtained in similar purity to the material described in Example 1 which may be determined by conventional methods such as HPLC and significant IR bands (Figure 1) at about 513, 538, 571, 592, 613, 665, 722, 761, 783, 806, 818, 839, 888, 906, 924, 947, 966, 982, 1006, 1034, 1068, 1091, 1134, 1194, 1221, 1248, 1286, 1340, 1387, 1493, 1513, 1562, 1604, 3402, 3631 cm⁻¹.

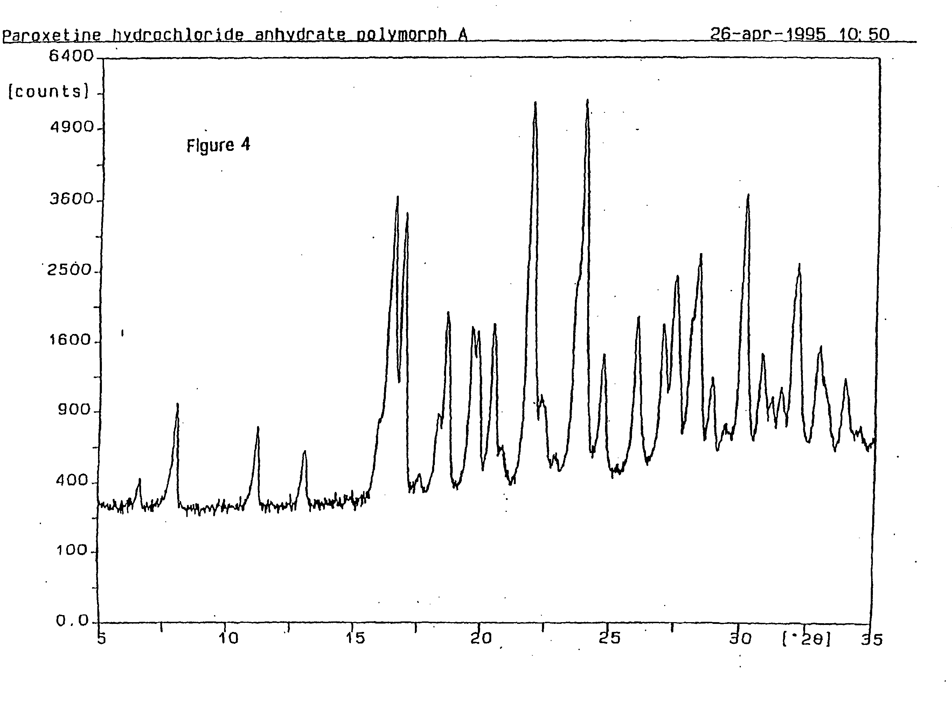

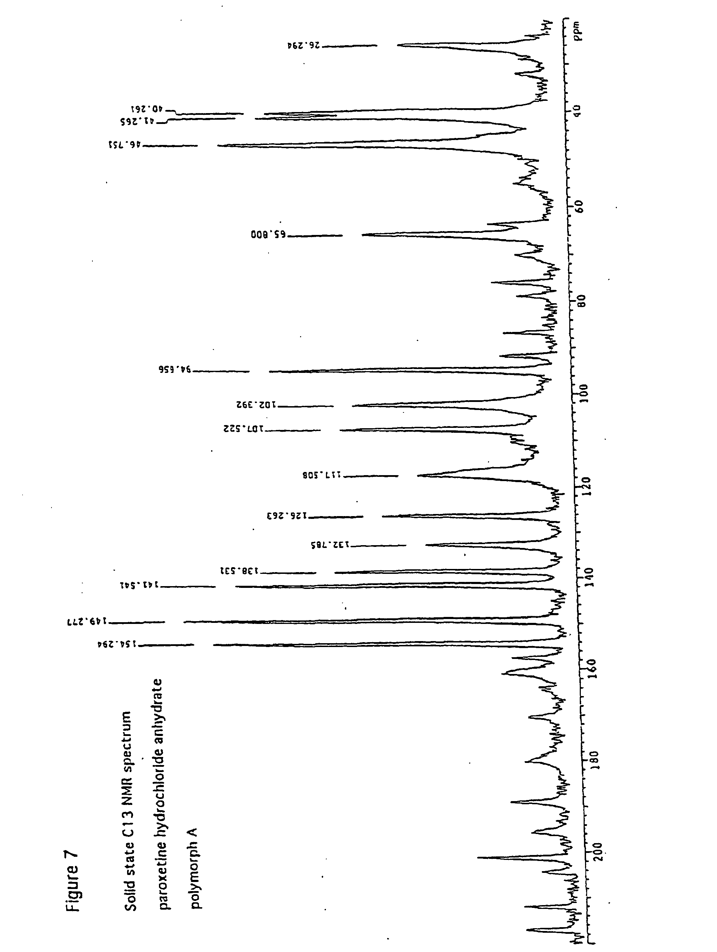

The DSC exotherm, measured at 10°C per minute shows a maximum at about 126°C using an open pan and a maximum at about 121°C using a closed pan. Form A also has a substantially similar X-ray diffractogram to that shown in Figure 4, for example there are characteristic peaks at 6.6, 8.0, 11.2, 13.1 degrees 2 theta and a substantially similar solid state NMR spectrum to that shown in Figure 7 for example with characteristic peaks at 154.3, 149.3, 141.6, 138.5 ppm.

The question of which particular form a particular sample of paroxetine hydrochloride anhydrate is would be readily determined by one skilled in the art using conventional techniques with reference to the data provided above that given in the examples and any other conventional means.

Preferably forms A exists as needles.

The present application also describes a process for the preparation of the paroxetine hydrochloride solvates other than the propan-2-ol solvate which comprises crystallising paroxetine hydrochloride in an organic solvent or mixture of solvents which form a solvate with the paroxetine hydrochloride and which are not removable by conventional drying techniques.

In first aspect, the present invention provides a process for the preparation of paroxetine hydrochloride anhydrate comprising less than 2 % bound organic solvent which comprises displacing the solvated solvent from a paroxetine hydrochloride solvate using a displacing agent, wherein the solvated solvent is acetone and the displacing agent is water.

In one preferred aspect of the invention crystallisation of paroxetine hydrochloride anhydrate is achieved by contacting a solution of paroxetine free base in an organic solvent or solvents with dry hydrogen chloride gas.

Alternatively, prior to the crystallisation of the paroxetine hydrochloride water may be removed by azeotropic distillation.

Thus, in another aspect of the invention paroxetine hydrochloride anhydrate is crystallised by dissolving paroxetine hydrochloride hemi-hydrate in an appropriate solvent substantially free of water which forms an azeotrope with water. Suitably solvent is removed by distillation and fresh solvent substantially free of water is added until all of the water is removed.

Paroxetine hydrochloride hemi-hydrate or the free base thereof may be prepared according to the procedures generally outlined in EP-B-0 223403.

The organic solvents should be substantially free of water to the extent that there is insufficient water present at the time of crystallisation to effect conversion to the hydrochloride hemi-hydrate. Organic solvents which are substantially free of water may be obtained in conventional manner. For example they can be dried using conventional techniques such as drying over molecular sieves or they can be purchased.

Factors which affect which form of the product will be obtained include the particular choice of organic solvent or solvents to be used will depend upon the particular form of the product which is desired.

It should also be appreciated that the method of solvent removal also depends upon the particular form of the product which is desired.

The paroxetine hydrochloride solvate produced by the process of the first aspect of the invention is suitably isolated and dried by conventional methods such as drying in vacuo to remove some or all of the free or unbound solvent. It should be appreciated that it is preferable and unexpected that the degree of drying is controlled such that only free solvent is removed. The bound solvent is then displaced with a displacing agent such as water. It is possible to use other displacing agents which may be selected by means of routine experimentation.

Preferably gaseous or liquid water may be used as a displacing agent. It is important that the paroxetine hydrochloride solvate is contacted with enough water and for sufficient time to displace the solvent but insufficient to cause conversion to the hydrochloride hemi-hydrate.

The amount of water, the form of the water, eg, liquid or gaseous and the length of time which the paroxetine hydrochloride solvate is contacted with the water differs from solvate to solvate. This depends largely upon the solubility of the solvate in question.

Particular ratios of paroxetine hydrochloride solvate to water are outlined in the examples hereinafter described (Examples 1, and 2). The use of the common ion effect when using diluted hydrochloric acid may help prevent dissolution of the solvate and subsequent conversion to the hydrochloride hemi-hydrate.

After contact with water to displace the bound solvent the product is suitably dried, for example, in vacuo at elevated temperature. Suitable drying may be over a desiccant such as phosphorus pentoxide.

The process of the invention is preferably used to prepare Form A.

Preferably the crystallisation of the paroxetine hydrochloride anhydrate Form A precursor solvate may be facilitated by the addition of seeds of paroxetine hydrochloride anhydrate Form A precursor solvate.

Alternatively, seeds of paroxetine hydrochloride anhydrate Form A may be used to facilitate the crystallisation of paroxetine hydrochloride anhydrate Form A precursor solvates.

Seed crystals of Form A may be prepared according to the procedures described herein or are freely available upon request to Corporate Intellectual Property, SmithKline Beecham plc at New Frontiers Science Park, Third Avenue, Harlow, Essex, CM19 5AW, United Kingdom. Form A is BRL 29060F.

In the following, examples 1 and 2 illustrate the present invention:

### Reference Example.

### Crystalline Paroxetine Hydrochloride Anhydrate Substantially Free of Bound Propan-2-ol (Form A)

### i) Paroxetine hydrochloride propan-2-ol solvate

Paroxetine hydrochloride hemihydrate [150 g] was stirred with propan-2-ol [1000 ml] and toluene [300 ml] in a round bottom flask and heated to boiling. Solvent was removed by distillation, the total volume being maintained by adding fresh propan-2-ol, until the boiling point had reached approximately 82°C, indicating that all the water had been removed.

The mixture was allowed to cool to approximately 50°C, when it spontaneously crystallised. The contents of the flask rapidly set to a thick paste which was diluted with propan-2-ol [approx. 500 ml] and stirred vigorously. The resulting suspension was allowed to cool to approximately 30°C and filtered under vacuum, taking care to avoid the absorption of atmospheric moisture. The solvent-wet cake was dried in high vacuum over phosphorus pentoxide.

Yield of solvated paroxetine hydrochloride 151 g, propan-2-ol content 13.0% (estimated by NMR).

The infra-red spectrum (Nujol mull) showed *inter alia* a characteristic band at 667 cm⁻¹.

### ii) Paroxetine hydrochloride anhydrate (Form A)

Paroxetine hydrochloride propan-2-ol solvate [110 g, propan-2-ol content 13.0%] was stirred in a beaker with water [275 ml] for 20 minutes. The mixture was filtered under vacuum and the damp solid dried in vacuum over phosphorus pentoxide to constant weight.

Yield of paroxetine hydrochloride anhydrate Form A 91.0 g

Water content 0.13% (KF), propan-2-ol content 0.05% (estimated by NMR).

Melting point: 123-125°C

The DSC exotherm, measured at 10°C per minute, showed a maximum at about 126°C using an open pan and a maximum at about 121°C using a closed pan.

The infra-red spectrum [Nujol mull] showed *inter alia* characteristic bands at 665, 3631 and 3402 cm⁻¹ (see Figure 1).

| Elemental analysis: | | | |
|---|---|---|---|
| Requires for paroxetine hydrochloride anhydrate | C 62.38 | H 5.79 | N 3.83% |
| Found | C 62.10 | H 5.89 | N 3.67% |

The sample was also examined by X-ray powder diffraction (see Figure 4) and solid state C13 NMR (see Figure 7).

### Example 1

### Paroxetine Hydrochloride Anhydrate Substantially Free of Bound Acetone (Form A)

### i) Paroxetine Hydrochloride Acetone solvate

Paroxetine free base (10.51 g) was dissolved in acetone (40 ml, dried with 4A molecular sieves), and a solution of hydrogen chloride gas (1.31 g) in dry acetone (10 ml) added with stirring. Crystallisation occurred spontaneously within one minute, and the mixture quickly became unstirrable. After approximately half an hour the product was filtered, placed in a desiccator over phosphorus pentoxide, and dried at ambient temperature overnight.

Weight of product: 11.24 g. Acetone content (estimated by NMR) 4% wt/wt. The infra-red spectrum showed a characteristic band at 667 cm⁻¹.

Approximately half the product was placed in a vacuum oven set at 50°C and dried further to constant weight. NMR analysis of the resulting product indicated the presence of 1.2% acetone wt/wt.

### ii) Paroxetine Hydrochloride Anhydrate (Form A)

A sample of the acetone solvate (5.18 g) was stirred for 10 minutes in water (20 ml), filtered, and dried at 50°C in a vacuum oven containing phosphorus pentoxide.

Weight of product: 4.63 g. NMR analysis indicated the presence of 0.6% acetone wt/wt. The infra-red spectrum corresponded to the spectrum of paroxetine hydrochloride anhydrate Form A and showed a characteristic band at 665 cm⁻¹.

### Example 2.

### Paroxetine Hydrochloride Anhydrate Substantially Free of Bound Acetone (Form A)

### i) preparation of paroxetine hydrochloride acetone solvate

A suspension of paroxetine hydrochloride anhydrate Form C (prisms) (5.0 g) in acetone (75 ml) was heated to boiling to give a mass of fine needles. The flask was sealed and allowed to stand overnight at room temperature. The solvent was removed at low temperature using a rotary evaporator and replaced by hexane (100 ml). The solvent was again removed at low temperature to give the acetone solvate as a crystalline residue. Analysis by NMR showed the presence of acetone (12.2% by weight), and the IR spectrum (Nujol mull) showed characteristic bands at 667 and 1714 cm -1.

### ii) Preparation of paroxetine hydrochloride anhydrate (Form A) from the acetone solvate

Paroxetine hydrochloride Form C (5.3 g) was converted to the acetone solvate by a similar procedure to that described above. Water (50 ml) was added, and the resulting suspension shaken gently for 10 minutes. The white solid was collected by filtration, drained thoroughly and dried in a vacuum oven at 50 °C. Yield 4.60 g. Acetone content (NMR) 0.1% by weight. The IR spectrum (Nujol mull) conformed to that of a standard sample of paroxetine hydrochloride anhydrate Form A.

## Claims

1. A process for the preparation of paroxetine hydrochloride anhydrate comprising less than 2% bound organic solvent which comprises displacing the solvated solvent from a paroxetine hydrochloride solvate using a displacing agent, wherein the solvated solvent is acetone , and the displacing agent is water.

## Patentansprüche

1. Verfahren zur Herstellung von Paroxetinhydrochloridanhydrat, umfassend weniger als 2% gebundenes organisches Lösungsmittel, welches Verdrängen des solvatisierten Lösungsmittels aus einem Paroxetinhydrochloridsolvat unter Verwendung eines Verdrängungsmittels umfasst, wobei das solvatisierte Lösungsmittel Aceton ist und das Verdrängungsmittel Wasser ist.

## Revendications

1. Procédé pour la préparation de chlorhydrate de paroxétine anhydre, comprenant moins de 2% de solvant organique lié, qui comprend le déplacement du solvant solvaté d'un produit de solvatation de chlorhydrate de paroxétine en utilisant un agent de déplacement, dans lequel le solvant solvaté est l'acétone et l'agent de déplacement est l'eau.
